# EUROPEAN PATENT SPECIFICATION

(11) **EP 2 510 918 B1**
(45) Date of publication and mention of the grant of the patent: **20.09.2017**
(21) Application number: 10836067.8
(22) Date of filing: 10.12.2010
(51) Int. Cl.: A61K 8/37, A61K 8/891, A61Q 1/04, A61K 8/39, A61Q 1/06, A61K 8/58, A61K 8/84

(54) **LIP COSMETICS**
LIPPENKOSMETIKARTIKEL
COSMÉTIQUE POUR LES LÈVRES

(30) Priority: 11.12.2009 JP 2009281560; 15.07.2010 JP 2010160611; 15.07.2010 JP 2010160614
(43) Date of publication of application: 17.10.2012
(73) Proprietor: SHISEIDO COMPANY, LTD., Tokyo 104-0061 (JP)
(72) Inventor: OSAWA Tomo, Yokohama-shi Kanagawa 224-8558 (JP); IKEDA Tomoko, Yokohama-shi Kanagawa 224-8558 (JP); TSUJI Yoshiharu, Yokohama-shi Kanagawa 224-8558 (JP); WATANABE Tomoko, Yokohama-shi Kanagawa 224-8558 (JP); TOMITA Noriko, Yokohama-shi Kanagawa 224-8558 (JP)
(74) Representative: Henkel, Breuer & Partner
(86) International application number: PCT/JP2010/072231
(87) International publication number: WO 2011/071148

(56) References cited:
- WO-A1-97/25960
- WO-A1-2005/046625
- WO-A2-2009/026113
- JP-A- 2005 068 027
- JP-A- 2005 247 739
- JP-A- 2005 314 370
- JP-A- 2006 282 592
- JP-A- 2008 247 804
- DATABASE WPI Week 200637 Thomson Scientific, London, GB; AN 2006-356902 XP002740178, & JP 2006 131563 A (SHISEIDO CO LTD) 25 May 2006 (2006-05-25)
- DATABASE WPI Week 200842 Thomson Scientific, London, GB; AN 2008-G62358 XP002740179, & JP 2008 019200 A (KOKYU ARUKORU KOGYO KK) 31 January 2008 (2008-01-31)
- DATABASE WPI Week 200929 Thomson Scientific, London, GB; AN 2009-G76645 XP002740180, & JP 2009 073797 A (SHISEIDO CO LTD) 9 April 2009 (2009-04-09)
- DATABASE WPI Week 200971 Thomson Scientific, London, GB; AN 2009-P75626 XP002740181, & JP 2009 234992 A (KOSE KK) 15 October 2009 (2009-10-15)
- DATABASE WPI Week 200680 Thomson Scientific, London, GB; AN 2006-785806 XP002740182, & JP 2006 282592 A (NARISU KESHOHIN KK) 19 October 2006 (2006-10-19)

## Description

### RELATED APPLICATIONS

This application claims the priority of Japanese Patent Application No. 2009-281560 filed on December 11, 2009, Japanese Patent Application No. 2010-160611 filed on July 15, 2010, and Japanese Patent Application No. 2010-160614 filed on July 15, 2010.

### FIELD OF THE INVENTION

The present invention relates to a lip cosmetic, and in particular, relates to a lip cosmetic having excellent secondary adhesion resistance effect immediately after the application and being excellent in gloss durability and stability.

### BACKGROUND OF THE INVENTION

Conventional lip cosmetics have presented the problem of secondary adhesion, namely a lipstick is transferred onto a site contacted by a lip (for example, a cup) after the lipstick is applied to the lip. By contrast, lip cosmetics having so-called secondary adhesion resistance effect that causes little secondary adhesion have been developed.

For example, Patent Document 1 discloses a transfer-resistant cosmetic composition comprising: a volatile hydrocarbon solvent; a non-volatile silicone compound that can be dissolved or dispersed in the volatile hydrocarbon solvent; and non-volatile hydrocarbon oil that is dissolved in the volatile solvent and is incompatible with the non-volatile silicone compound, wherein the non-volatile hydrocarbon oil has a certain solubility parameter.

However, this transfer-resistant cosmetic composition has room for improvement in stability. Due to its large amount of wax, the feeling in use in a liquid state cannot be obtained, and also gloss is insufficient.

Patent Document 2 discloses a lipstick composition having transfer resistance, comprising perfluoropolyether-type non-volatile oil and volatile oil, which are incompatible with each other. In this Patent Literature 2, oils are separated during application to a support to move onto a first composition.

However, the first composition is in a solid state due to a considerable amount of wax. Thus, a sufficient gloss or moisture cannot be obtained. Moreover, for this system, the incompatible oil phases are difficult to be favorably dispersed, resulting in the problem of stability against sweating etc.

Patent Document 3 discloses a stick cosmetic having transfer resistance, comprising volatile oil and a silicone surfactant, wherein pigments are favorably dispersed.

However, this stick cosmetic has a large proportion of the volatile oil in the composition and thus has the disadvantage that its matte finish tends to provide a feeling of dryness on lips.

Patent Document 4 discloses a one-phase composition for lipsticks, comprising volatile oil and a silicone resin.

However, after evaporation of the volatile oil, this composition for lipsticks tends to cause a feeling of dryness over time, although it has improved transfer resistance. Moreover, a film of the resin remains on lips. The composition further has the following disadvantages that; it causes a filmy feeling and tightness, and the obtained adhesion is matte.

Patent Document 5 discloses an oil-in-oil emulsion composition comprising: continuous-phase oil comprising a silicone coating agent, volatile silicone oil, non-volatile silicone liquid oil, and an emulsifying agent; and dispersion-phase oil comprising ester oil and a coloring material, wherein the blending quantities of the continuous-phase oil and the dispersion-phase oil are at a dispersion-phase oil/(dispersion-phase oil and continuous-phase oil) ratio of 0.05 to 0.5.

However, this oil-in-oil emulsion composition tends to generate color unevenness due to the presence of the coloring material in the dispersion phase. Furthermore, for this system, temporal stability may be difficult to maintain.

Patent Document 6 discloses a cosmetic composition that may be in the form of a lip makeup product, the cosmetic composition comprising about 3 to 35% by mass of a styrene/acrylate copolymer, about 1 to 35% by mass of a siloxane such as phenyl trimethicone, about 1 to 35% by mass of an ester such as polyglyceryl-4 isostearate, and about 0.1 to 20% by mass of a rheology agent. In a preferred embodiment, the rheology agent comprises a wax.

Patent Document 7 discloses a lipstick composition having good transfer-resistance, the lipstick composition comprising 30 to 95% by mass of a lipophilic material selected from the group consisting of waxes, oils and mixtures thereof, and 1 to 70% by mass of a volatile fluid selected from the group consisting of volatile hydrocarbons, volatile silicones and mixtures thereof. In an especially preferred embodiment, the volatile silicone is a cyclomethicone.

Patent literature 1: Japanese unexamined patent publication No. 2001-199846
Patent literature 2: International unexamined patent publication No. 96/40044
Patent literature 3: International unexamined patent publication No. 97/16157
Patent literature 4: Japanese unexamined patent publication No. H9-48709
Patent literature 5: Japanese unexamined patent publication No. 2000-53530
Patent literature 6: International unexamined patent publication No. 2009/026113
Patent literature 7: International unexamined patent publication No. 97/25960

### DISCLOSURE OF THE INVENTION

### PROBLEM TO BE SOLVED BY THE INVENTION

The present invention was made in view of the above-described conventional art. An object of the invention is to provide a lip cosmetic that has excellent secondary adhesion resistance effect immediately after the application and is excellent in gloss durability after application and stability.

### MEANS TO SOLVE THE PROBLEM

The present inventors have diligently studied; as a result, the present inventors have found that a stable lip cosmetic having, after application, both secondary adhesion resistance and a gloss can be obtained by using a specific surfactant and specific oil(s).

In one aspect, the present invention thus relates to a lip cosmetic which is characterized by comprising the following (a) and (b), wherein the blending quantity of (b) is 20 to 70 mass %:
(a) 5 to 20 mass % of a polyglyceryl isostearate wherein the addition mole number of glycerin is 4 to 10, and the number of isostearic acid residues is 1 to 4; and
(b) (b1) 20 to 70 mass % of one or more kinds of methyl phenyl silicones, wherein said one or more kinds of methyl phenyl silicones are selected from the group consisting of trimethyl pentaphenyl trisiloxane, diphenyl dimethicone and diphenylsiloxyphenyl trimethicone, (b2) 0 to 45 mass % of a pentaerythritol ester,
wherein, when component (b1) contains diphenylsiloxy phenyl trimethicone and component (b2) is absent, the blending quantity of diphenylsiloxy phenyl trimethicone in component (b1), as defined by the equation diphenylsiloxy phenyl trimethicone/{component (b1) other than diphenylsiloxy phenyl trimethicone}, is 0.8 to 3.5 (mass ratio).

In the lip cosmetic, it is preferable that component (b1) as a whole does not separate when mixed with component (a) at 130 °C and separates when mixed with component (a) at 25 °C.

In the lip cosmetic, it is preferable that (c) 4 to 10 mass % of a wax is contained.

In the lip cosmetic, it is preferable that component (b1) contains diphenylsiloxy phenyl trimethicone.

In the lip cosmetic comprising diphenylsiloxy phenyl trimethicone in (b1), it is preferable that, when component (b2) is present, the blending quantity of diphenylsiloxy phenyl trimethicone in component (b1), as defined by the equation diphenylsiloxy phenyl trimethicone/{other component (b1) other than diphenylsiloxy phenyl trimethicone}, is 0.4 to 3.5 (mass ratio).

In the lip cosmetic, it is preferable that (d) glycerin is contained.

In the lip cosmetic, it is preferable that component (a) contains 40 mass % or higher polyglyceryl isostearate, which has a degree of polymerization of 5, in the total amount of component (a).

In the lip cosmetic, it is preferable that component (a) is polyglyceryl-5 triisostearate.

In another aspect, the present invention relates to a lip cosmetic which is characterized by comprising the following (a) and (b), wherein the blending quantity of (b) is 30 to 70 mass %:
(a) 5 to 20 mass % of a polyglyceryl isostearate wherein the addition mole number of glycerin is 4 to 10, and the number of isostearic acid residues is 1 to 4; and
(b) (b1) 20 to 60 mass % of one or more kinds of methyl phenyl silicones, wherein said one or more kinds of methyl phenyl silicones are selected from the group consisting of trimethyl pentaphenyl trisiloxane, diphenyl dimethicone and diphenylsiloxyphenyl trimethicone, (b2) 5 to 45 mass % of a pentaerythritol ester.

In still another aspect, the present invention relates to a lip cosmetic which is characterized by comprising the following (a) to (c):
(a) 5 to 20 mass % of a polyglyceryl isostearate wherein the addition mole number of glycerin is 4 to 10, and the number of isostearic acid residues is 1 to 4;
(b) 20 to 70 mass % of one or more kinds of methyl phenyl silicones that does not separate when mixed with component (a) at 130 °C and separate when mixed with component (a) at 25 °C, wherein said one or more kinds of methyl phenyl silicones are selected from the group consisting of trimethyl pentaphenyl trisiloxane, diphenyl dimethicone and diphenylsiloxyphenyl trimethicone; and
(c) 5 to 10 mass % of a wax,
wherein, when component (b) contains diphenylsiloxy phenyl trimethicone and the lip cosmetic does not comprise a pentaerythritol ester, the blending quantity of diphenylsiloxy phenyl trimethicone in component (b), as defined by the equation diphenylsiloxy phenyl trimethicone/{component (b) other than diphenylsiloxy phenyl trimethicone}, is 0.8 to 3.5 (mass ratio).

### EFFECT OF THE INVENTION

A lip cosmetic having good gloss and good stability, while maintaining secondary adhesion resistance effect, can be obtained by blending the specific amounts of (a) specific polyglyceryl isostearate, and (b) one or more kinds of methyl phenyl silicones selected from the group consisting of trimethyl pentaphenyl trisiloxane, diphenyl dimethicone and diphenylsiloxyphenyl trimethicone, and/or pentaerythritol ester.

### BEST MODE FOR CARRYING OUT THE INVENTION

Generally, if the secondary adhesion resistance effect is high, the gloss upon application has a tendency to be lacking. On the other hand, the base having a gloss has a drawback in that the secondary adhesion easily takes place because there is plenty residual oil. In the present invention, by using a specific surfactant and specific oil(s) (silicone oil and/or ester oil), the oil separates into the surface layer and a gloss is provided. Because the surfactant, in the inner layer, takes in the coloring material, the secondary adhesion is difficult to take place. As a result, a lip cosmetic without secondary adhesion and with an excellent gloss can be obtained.

On this occasion, the dispersion phase has high adhesiveness; therefore, the oil separates to the surface layer immediately upon application, and the secondary adhesion resistance effect is exhibited right away.

In the following, each component is described in detail.

### ((a) Polyglyceryl isostearate)

The polyglyceryl isostearate used in the present invention as component (a) is a surfactant, and it is obtained by adding isostearic acid to a polyglycerin having the average addition mole number of 4 to 10 without specifying the addition locations. In particular, a polyglycerin having the average addition mole number of 5 is preferable. In addition, it is necessary to use a polyglyceryl isostearate wherein 1 to 4 isostearic acid(s) are added in one molecule, and a polyglyceryl isostearate containing 2 to 4 isostearic acids is especially preferable.

In the present invention, component (a) takes in the coloring material, by contact during application, and swiftly separates from component (b).

The polyglyceryl isostearate can be provided by various publicly known synthesis methods. However, the one having a narrow distribution of the glycerin addition mole number and containing less cyclic compounds as impurities is preferable.

The polyglyceryl isostearate can be produced, for example, by the method described in Japanese Patent No. 3487881 and Japanese Unexamined Patent Publication No. 2006-111539 (a polyglycerin fatty acid ester obtained by the esterification of a fatty acid and a polyglycerin that has a hydroxyl value of 1200 or less and has 50% or more primary hydroxyl groups relative to all the hydroxyl groups).

It is especially preferable that the content of polyglyceryl-5 isostearate, wherein the degree of polymerization is 5 in the polyglyceryl isostearate, is 40 mass % or higher in the total amount of component (a).

This polyglyceryl-5 isostearate can be provided by various publicly known synthesis methods. In the present invention, it is preferable to use polyglyceryl-5 isostearate that is obtained by using isostearic acid and polyglycerin-5, wherein the content of polyglycerins with a low degree of polymerization is small and the distribution of the degree of polymerization is narrow, as the raw materials. In particular, it is preferable that the content of polyglyceryl-5 isostearate, wherein the degree of polymerization of glycerin is 5, is 40 mass % or higher in the total amount of component (a).

Furthermore, polyglyceryl-5 triisostearate wherein the number of isostearic acid residues is 2 to 4 is preferable, and polyglyceryl-5 triisostearate wherein the number of isostearic acid residues is 3 is especially preferable.

According to a normal synthesis method, when a polyglycerin is produced using glycerin as the raw material, numerous undesirable by-products, by intramolecular condensation and the formation of 6-membered rings and 8-membered rings, are generated on the occasion of dehydrating condensation. Therefore, so that these by-products are not produced, a polyglycerin that can be obtained by the synthesis and purification using glycidol, epichlorohydrin, monochlorohydrin, etc. as the raw material is preferable. When the polyglycerin and a fatty acid are reacted, a polyglycerin with a low molecular weight normally has a higher reactivity with a fatty acid compared with a polyglycerin with a high molecular weight. As a result, when a polyglycerin having a wide molecular weight distribution is used as the raw material, a uniform ester cannot be produced. Accordingly, a polyglycerin having a molecular weight distribution as narrow as possible is preferable. For example, a polyglycerin can be obtained by a dehalogenation alkali metal salt reaction using glycerin or a partial alcoholate of its polymer and a halogenated hydrocarbon or an oxyhalogenated hydrocarbon as the raw materials.

The production method is illustrated by an example. In the first process, diglycerin monoalcoholate is formed by adding 1 mole of sodium hydroxide to 1 mole of diglycerin and dehydrating by heating. In the second process, 1 mole of dichlorohydrin is added to 2 moles of the obtained diglycerin monoalcoholate and heated. Then, 1 mole of a narrowly-distributed polyglycerin with the degree of polymerization of 5 can be obtained.

The detailed preparation method of a narrowly-distributed polyglycerin is described, for example, in Japanese Patent No. 3487881.

Polyglyceryl-5 isostearate of the present invention is obtained by the esterification of the above-obtained polyglycerin with isostearic acid by a publicly known method. For example, the esterification can be carried out either in the presence of an alkaline catalyst or acid catalyst or in the absence of a catalyst under either ordinary pressure or reduced pressure.

The blending quantity of (a) polyglyceryl isostearate of the present invention is 5 to 20 mass % and preferably 12 to 20 mass %. If the blending quantity of component (a) is too large or too small, the secondary adhesion resistance effect will be poor. In addition, if it is too large, stickiness after application tends to be generated.

### ((b) Methyl phenyl silicone and/or pentaerythritol ester)

Component (b) used in the present invention separates from component (a) after application and forms the surface layer. Thus, the secondary adhesion resistance effect is exhibited and the gloss can be improved.

In the component (b) of the present invention, component (b1), namely methyl phenyl silicones are essential components; component (b2), namely a pentaerythritol ester is not an essential component. However, a lip cosmetic having a better secondary adhesion resistance effect and stability can be obtained by combining component (b1) and component (b2).

The (b1) methyl phenyl silicone can be one kind or a mixture of two or more kinds. To satisfy the secondary adhesion resistance effect, it is preferable that component (b1) as a whole does not separate when mixed with (a) at 130 °C and separates when mixed with (a) at 25 °C.

Here, the presence or absence of "separation" was measured under the following conditions.

### (Measurement condition)

(a) and (b1) were used in the ratio ((a):(b1) = 1:3 (mass ratio)). In the case that the mixture was heated to 130 °C and mixed with stirring or after that it was allowed to stand at 25 °C, when the boundary was uniformly separated into two layers, it was denoted "separated". When it was non-uniformly and cloudy state or a translucent state or a transparently miscible state without a boundary, it was denoted "not separated".

According to the invention, trimethyl pentaphenyl trisiloxane, diphenyl dimethicone, and diphenylsiloxy phenyl trimethicone are used as the (b1) methyl phenyl silicone.

Furthermore, it is preferable to blend methyl phenyl silicones at the percentages that satisfy the above-described conditions as a whole.

As a commercial trimethyl pentaphenyl trisiloxane, methyl phenyl silicone FZ3156 (165 mm²/s (25 °C), manufactured by Dow Corning Toray Co., Ltd.) can be listed. As a commercial diphenyl dimethicone, silicone KF54 (400 mm²/s (25 °C), manufactured by Shin-Etsu Chemical Co., Ltd.), silicone KF50-300CS (manufactured by Shin-Etsu Chemical Co., Ltd.), silicone KF-54HV (manufactured by Shin-Etsu Chemical Co., Ltd.), and the like can be listed. Examples of diphenylsiloxy phenyl trimethicones include silicone KF56 (14 mm²/s (25 °C), manufactured by Shin-Etsu Chemical Co., Ltd.)).

The blending quantity of (b1) methyl phenyl silicone is 20 to 70 mass %, preferably 25 to 60 mass %, and especial preferably 30 to 55 mass %. If the blending quantity of component (b1) is less than 20 mass %, the secondary adhesion easily takes place and there is a little gross. If it exceeds 70 mass %, the stability is poor.

In the present invention, diphenylsiloxy phenyl trimethicone is preferably blended as component (b1). The blending quantity of diphenylsiloxy phenyl trimethicone is preferably 10 to 50 mass %.

It is preferable to allow the blending quantity of diphenylsiloxy phenyl trimethicone in component (b1) to be in the ratio, diphenylsiloxy phenyl trimethicone/{other component (b1) other than diphenylsiloxy phenyl trimethicone} = 0.4 to 3.5 (mass ratio), to satisfy both the secondary adhesion resistance effect and stability. Diphenylsiloxy phenyl trimethicone/{other component (b1) other than diphenylsiloxy phenyl trimethicone} is more preferably 0.5 to 3.5 (mass ratio) and especial preferably 1.2 to 2 (mass ratio). When component (b2) is not blended, the ratio diphenylsiloxy phenyl trimethicone/{other component (b1) other than diphenylsiloxy phenyl trimethicone} = 0.8 to 3.5 (mass ratio).

It is possible to produce (b2) a pentaerythritol ester by a common synthesis method. For example, into a suitable reaction container, (di)pentaerythritol and acids such as benzoic acid, 2-ethylhexanoic acid, and behenic acid, which are the acids corresponding to the product compound, are placed (the addition order is not limited in particular). The compound can be obtained by reacting them either in the presence or absence of acid, alkaline, or other metal catalysts, and preferably in an organic solvent or/and gas, which is inert to the reaction, at 150 to 250 °C for several hours to about 30 hours while removing the by-product water.

Examples of pentaerythritol esters of the present invention include pentaerythrityl tetra(benzoate/2-ethylhexanoate), pentaerythrityl tetra(behenate/benzoate/2-ethylhexanoate), pentaerythrityl tetra-2-ethylhexanoate, and dipentaerythrityl hexa-12-hydroxystearate. One or more kinds of such pentaerythritol esters can be used.

Among them, a liquid pentaerythritol ester can be suitably used because it is less likely to be separated when mixed with component (a) and component (b1) at 130 °C.

Examples of liquid pentaerythritol esters include pentaerythrityl tetra(benzoate/2-ethylhexanoate) and pentaerythrityl tetra-2-ethylhexanoate.

The blending quantity of (b2) pentaerythritol ester is 0 to 45 mass %, preferably 5 to 45 mass %, and especial preferably 10 to 40 mass %. If the blending quantity of component (b2) is too small, the secondary adhesion may easily take place and there may be a little gross. If it is too large, the stability may be poor.

In the present invention, the blending quantities of component(s) (b) (namely, the total blending quantities of (b1) and (b2)) are 20 to 70 mass % and preferably 50 to 70 mass %. If the blending quantity of component (b) is less than 20 mass %, the secondary adhesion easily takes place and there is a little gross. If it exceeds 70 mass %, the stability becomes poor.

### ((c) Wax)

In the present invention, in addition to the above-described essential components, it is preferable to further blend (c) wax.

Wax is not limited in particular as long as it can be normally blended for cosmetics, and the examples include carnauba wax, candelilla wax, beeswax, ceresin, microcrystalline wax, solid paraffin, Japan wax, and polyethylene wax.

The blending quantity of (c) wax is preferably 4 to 10 mass %, more preferably 5 to 10 mass %, and especial preferably 6 to 9 mass %. If the blending quantity of wax is too small, the solidification may be difficult. If it is too large, the spreadability may become heavy and the gloss may be lost.

### ((d) Glycerin)

In the present invention, it is preferable to further blend (d) glycerin. The secondary adhesion resistance effect is improved by blending component (d).

If a part of (a) polyglyceryl isostearate, for example, 20 to 40 mass % of the total amount of component (a) is replaced by glycerin, an association structure is formed, and the viscosity of the component that sticks to the lip is higher than the case in which only component (a) is used.

Because (d) glycerin is used as a raw material in the production of a polyglyceryl isostearate, it may be contained as an impurity in the polyglyceryl isostearate. Thus, it may not be necessary to separately add glycerin.

In the lip cosmetic of the present invention, in addition to the above-described components, the components normally used in lip cosmetics (for example, oil other than the above-described oils, powder, polymer compound, moisturizer, perfume, antioxidant agent, preservative, and beauty component) can be blended so far as the effect of the present invention is not undermined.

As the additional oil to the above-described components (b1) and (b2), for example, it is preferable to blend an oil that compatibilizes the whole system at 130 °C. The examples of such oils include isoparaffin.

Only the above-described component and essential components may be contained as the oil, and it is preferable not to contain other oils.

The examples of moisturizers include polyol moisturizers such as propylene glycol and 1,3-butylene glycol.

A coloring material can be blended in the lip cosmetic of the present invention. The secondary adhesion resistance effect can be markedly felt by blending a coloring material.

Such coloring materials can be powdery or lake-like (oil-containing state) so far as they are coloring materials normally used in lip cosmetics. They can be inorganic pigments, organic pigments, or pearlescent agents. The coloring material is taken in component (a) when the cosmetic is applied and it is present in the inner side of component (b); thus the secondary adhesion is difficult to take place.

The blending quantity of coloring material is preferably 1 to 13 mass % and especial preferably 3 to 8 mass %.

A film-forming agent can be blended in the lip cosmetic of the present invention.

Examples of film-forming agents include (alkyl acrylate/dimethicone) copolymer and the like. Specifically, silicone KP545 (manufactured by Shin-Etsu Chemical Co., Ltd.) and the like can be listed as a commercial product.

When a film-forming agent is blended, the blending quantity is preferably 2 to 15 mass % and especial preferably 5 to 10 mass %.

It is preferable that the lip cosmetic of the present invention is constituted so that the separation does not take place throughout the entire production process and the state of one homogeneous phase is maintained. More specifically, it is preferable that the lip cosmetic is constituted so that the entire composition does not separate at 130 °C and the state of one homogeneous phase is maintained.

The lip cosmetic of the present invention can be applied to lipsticks, lip glosses, lip bases, overcoats for lipsticks, lip creams, and the like.

### EXAMPLES

The present invention will be further described in the following examples. However, the invention is not limited by these examples. Unless otherwise specified, the blending quantity of each component will be expressed in mass %.

Prior to illustrating the examples, the test methods for the effects used in the present invention will be explained.

### Evaluation (1): Secondary adhesion resistance effect

The actual usability test by 10 professional panelists was carried out. The five-level sensory evaluation (scoring) of the secondary adhesion resistance effect upon application to the lip was based on the below-described scoring criteria. The determination was by the score average value based on the below-described evaluation criteria.

### (Score)

5 points: very excellent
4 points: excellent
3 points: ordinary
2 points: poor
1 point: very poor

### (Evaluation criteria)

S: The score average value is 4.7 points or higher and less than 5.0 points.
A*: The score average value is 4.5 points or higher and less than 4.7 points.
A: The score average value is 4 points or higher and less than 4.5 points.
B*: The score average value is 3.5 points or higher and less than 4 points.
B: The score average value is 2.5 points or higher and less than 3.5 points.
C: The score average value is 1.0 point or higher and less than 2.5 points.

The examples listed with "-" in the table had poor stability, and the secondary adhesion resistance effect could not be evaluated.

### Evaluation (2): Stability

The color uniformity of the cutting plane of the stick-shaped sample was evaluated based on the below-described evaluation criteria.

### (Evaluation criteria)

A*: It is uniform.
A: It is uniform; however, the coloration is poor.
B: It is slightly non-uniform.
C: It is non-uniform.

### Evaluation (3): Gloss

The actual usability test by 10 professional panelists was carried out. The five-level sensory evaluation (scoring) of the gloss upon application to the lip was based on the below-described scoring criteria. The determination was by the score average value based on the below-described evaluation criteria.

### (Score)

5 points: very excellent
4 points: excellent
3 points: ordinary
2 points: poor
1 point: very poor

### (Evaluation criteria)

S: The score average value is 4.7 points or higher and less than 5.0 points.
A*: The score average value is 4.5 points or higher and less than 4.7 points.
A: The score average value is 4 points or higher and less than 4.5 points.
B*: The score average value is 3.5 points or higher and less than 4 points.
B: The score average value is 2.5 points or higher and less than 3.5 points.
C: The score average value is 1.0 point or higher and less than 2.5 points.

### Evaluation (4): Evaluation test of the separation state of (a) and (b1)

Component (a) and component (b1) were mixed and measured under the following conditions. If the separation did not take place at 130 °C and the separation took place at 25 °C it was denoted as "A", and others were denoted as "C".

### (Measurement condition)

(a) and (b1) were used in the ratio ((a):(b1) = 1:3 (mass ratio)). In the case that the mixture was heated to 130 °C and mixed with stirring or after that it was allowed to stand at 25 °C, when the boundary was uniformly separated into two layers, it was denoted "separated". When it was non-uniformly and cloudy state or a translucent state or a transparently miscible state without a boundary, it was denoted "not separated".

### Production Example 1 (production of polyglyceryl-5 isostearate)

Into a 5 L four-neck flask, 3300 g of diglycerin and 800 g of 50% sodium hydroxide aqueous solution were placed, and heated to 140 °C while removing water under nitrogen stream. After the distillation of water was completed, 640 g of dichlorohydrin was dropwise added over the period of 2 hours. After the dropwise addition, it was stirred for 2 hours at 120 °C. From this, an excess amount of diglycerin was removed by molecular distillation. Then, it was diluted with water and decolorized and desalted with activated charcoal and ion exchange resin, and polyglycerin was obtained by the removal of water. This product was trimethylsilylated and analyzed by GC method; the amount of a component with the degree of polymerization of 5 was 60%.

The obtained polyglycerin-5 was reacted with isostearic acid by the conventional method, and a polyglyceryl-5 isostearate with various addition mole numbers of isostearic acid was obtained.

### Production Example 2 (production of polyglyceryl-10 isostearate)

Into a three-neck flask with a thermometer, a Dimroth condenser, and a stirring device, 200 g of polyglycerin manufactured by Taiyo Kagaku Co., Ltd. (Great Oil DE-1, decaglycerin; hydroxyl value: 890, percentage of the primary hydroxyl groups: 46.6%, percentage of the secondary hydroxyl groups: 53.4%) and 600 ml of pyridine were added. To this, 370 g of triphenylchloromethane (manufactured by Wako Pure Chemical Industries, Ltd.), which is a reagent that selectively reacts with a primary hydroxyl group, was added, stirred for 1 hour at 100 °C, returned to room temperature, and stirred for 24 hours. Then, most of the pyridine was removed from the reaction solution under reduced pressure. To the obtained product, 800 ml of water was added, transferred to a separatory funnel, and extracted three times with 400 ml of ethyl acetate. The ethyl acetate layers were combined and concentrated, 156 g of the obtained residue and 300 g of acetic acid were added into a three-neck flask with a thermometer, a Dimroth condenser, and a stirring device, and heated under reflux for 8 hours to eliminate a trimethylphenyl group. The above process was repeated, and purified polyglycerin was mixed to obtain a certain amount of polyglycerin. The hydroxyl value of the obtained polyglycerin was 886, the percentage of the primary hydroxyl groups was 61.3%, and the percentage of the secondary hydroxyl groups was 38.7%.

The hydroxyl value was calculated according to the "Test Methods for Oils and Fats" of the Japanese Standards of Food Additives, 7-th Ed. or the Standard Methods for the Analysis of Fats, Oils and Related Materials.

The percentages of the primary hydroxyl groups and the secondary hydroxyl groups were determined by spectral analysis with a nuclear magnetic resonance apparatus. That is, with the use of a nuclear magnetic resonance apparatus (13C-NMR) (manufactured by JEOL Ltd., JNM-A 500), the percentages of the primary hydroxyl groups and the secondary hydroxyl groups of the above fractionated polyglycerin were analyzed. Into 2.8 ml of heavy water, 500 mg of the fractionated polyglycerin was dissolved, and a 13C-NMR (125 MHz) spectrum was obtained by gated decoupling after filtration. The peak intensity in the gated decoupling measurement method is proportional to the number of carbon atoms. 13C chemical shifts that indicate the presence of the primary hydroxyl group and the secondary hydroxyl group were located near 63 ppm for the methylene carbon (CH₂OH) and near 71 ppm for the methine carbon (CHOH), respectively. By the analysis of the signal intensities of two respective species, the abundance percentages of the primary hydroxyl groups and the secondary hydroxyl groups were calculated. However, the methine carbon (CHOH), which shows the secondary hydroxyl group, overlaps the peak of the methylene carbon neighboring the methine carbon bonded to the methylene carbon, which shows the primary hydroxyl group. Thus, the integration value of the methine carbon itself cannot be obtained. Therefore, the integration value was calculated based on the intensity of the signal, near 74 ppm, of methylene carbon (CH₂) neighboring the methine carbon (CHOH).

The obtained polyglycerin-10 was reacted with isostearic acid by the conventional method, and a polyglyceryl-10 isostearate with various addition mole numbers of isostearic acid was obtained.

Samples (lipsticks) with the blending compositions shown in the below Tables 1 to 7 were produced by the ordinary method. Respective samples were evaluated, for the evaluation items (1) to (4), based on the above criteria. The results are shown in Tables 1 to 7.

**[Table 1]**

| Test Example | | 1-1 | 1-2 | 1-3 | 1-4 | 1-5 | 1-6 |
|---|---|---|---|---|---|---|---|
| (a) | Polyglyceryl-5 triisostearate 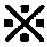 1 | 20 | - | - | - | - | - |
| | Polyglyceryl-10 triisostearate 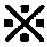 2 | - | 20 | - | - | - | - |
| | Polyglyceryl-5 isostearate3 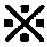 3 | - | - | 20 | - | - | - |
| | Polyglyceryl-5 isostearate(1.5 equivalence) 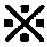 4 | - | - | - | 20 | - | - |
| | Polyglyceryl-5 diisostearate 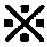 5 | - | - | - | - | 20 | - |
| | Polyglyceryl-5 tetraisostearate 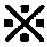 6 | - | - | - | - | - | 20 |
| (b1) | Diphenylsiloxy phenyl trimethicone 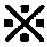 7 | 40 | 40 | 40 | 40 | 40 | 40 |
| | Trimethyl pentaphenyl trisiloxane 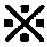 8 | 12.5 | 12.5 | 12.5 | 12.5 | 12.5 | 12.5 |
| | Diphenyl dimethicone 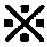 9 | 12.5 | 12.5 | 12.5 | 12.5 | 12.5 | 12.5 |
| (c) | Polyethylene wax | 8 | 8 | 8 | 8 | 8 | 8 |
| | Coloring material | 6 | 6 | 6 | 6 | 6 | 6 |
| | Hydrophobized pearlescent agent | 1 | 1 | 1 | 1 | 1 | 1 |
| Evaluation (1): Secondary adhesion resistance effect | | A | A | A | A | A | A |
| Evaluation (2): Stability | | A* | A* | A | A | A* | A* |
| Evaluation (3): Gloss | | A* | A* | A* | A* | A* | A* |
| Evaluation (4): Separation state of (a) and (b1) | | A | A | A | A | A | A |

| | | | | | | | |
|---|---|---|---|---|---|---|---|
| | | | | | | | |

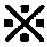 1: Material produced in Production Example 1. The average addition mole number of isostearic acid = 3.
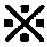 2: Material produced in Production Example 2. The average addition mole number of isostearic acid = 3.
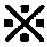 3: The average addition mole number of isostearic acid = 1.
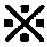 4: The average addition mole number of isostearic acid = 1.5.
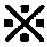 5: The average addition mole number of isostearic acid = 2.
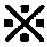 6: The average addition mole number of isostearic acid = 4.
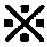 7: Silicone KF56 (manufactured by Shin-Etsu Chemical Co., Ltd., viscosity: 14 mPa·s)
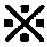 8: Methyl phenyl silicone FZ3156 (manufactured by Dow Corning Toray Co., Ltd., viscosity: 165 mPa·s)
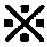 9: Silicone KF54 (manufactured by Shin-Etsu Chemical Co., Ltd., viscosity: 400 mPa·s)

In Test Examples 1-1 to 1-6, wherein a diverse polyglyceryl isostearate and various methyl phenyl silicones were blended, the secondary adhesion resistance effect and stability were excellent, and there was an excellent gloss.

According to Table 1 and the investigation of the present inventors, it is necessary that the addition mole number of glycerin is 4 to 10, and the number of isostearic acid residues is 1 to 4 in the polyglyceryl isostearate used in the present invention.

According to evaluation item (4) in Table 1, such a polyglyceryl isostearate and methyl phenyl silicones form a composite that does not separate at a high temperature and separates at an ordinary temperature.

That is, in the lip cosmetic, of the present invention, containing (a) a polyglyceryl isostearate and (b1) methyl phenyl silicones, it is preferable that component (b1) does not separate when mixed with (a) at 130 °C and separates when mixed with (a) at 25 °C.

**[Table 2]**

| Test Example | | 1-1 | 2-1 | 2-2 | 2-3 |
|---|---|---|---|---|---|
| (a) | Polyglyceryl-5 triisostearate 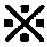 1 | 20 | 16 | 14 | 12 |
| (b1) | Diphenylsiloxy phenyl trimethicone 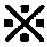 7 | 40 | 40 | 40 | 40 |
| | Trimethyl pentaphenyl trisiloxane 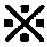 8 | 12.5 | 12.5 | 12.5 | 12.5 |
| | Diphenyl dimethicone 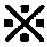 9 | 12.5 | 12.5 | 12.5 | 12.5 |
| (c) | Polyethylene wax | 8 | 8 | 8 | 8 |
| (d) | Dynamite glycerin | - | 4 | 6 | 8 |
| | Coloring material | 6 | 6 | 6 | 6 |
| | Hydrophobized pearlescent agent | 1 | 1 | 1 | 1 |
| Evaluation (1): Secondary adhesion resistance effect | | A | A* | S | S |
| Evaluation (2): Stability | | A* | A* | A* | A* |
| Evaluation (3): Gloss | | A* | A* | A* | A* |
| Evaluation (4): Separation state of (a) and (b1) | | A | A | A | A |

According to Table 2, it is seen that if a part of polyglyceryl isostearate is replaced with glycerin in the sample of Test Example 1-1, wherein a polyglyceryl isostearate and methyl phenyl silicones are suitably blended, the secondary adhesion resistance effect improves.

Accordingly, (d) glycerin is preferably blended in the lip cosmetic of the present invention.

**[Table 3]**

| Test Example | | 3-1 | 3-2 | 2-2 | 3-3 | 3-4 | 3-5 | 3-6* | 3-7 |
|---|---|---|---|---|---|---|---|---|---|
| (a) | Polyglyceryl-5 triisostearate 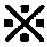 1 | 14 | 14 | 14 | 14 | 14 | 14 | 14 | 14 |
| (b1) | Diphenylsiloxy phenyl trimethicone 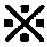 7 | 50 | 50 | 40 | 40 | 40 | 30 | 20 | 10 |
| | Trimethyl pentaphenyl trisiloxane 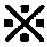 8 | - | 7.5 | 12.5 | 25 | - | 17.5 | 22.5 | 25 |
| | Diphenyl dimethicone 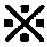 9 | 15 | 7.5 | 12.5 | - | 25 | 17.5 | 22.5 | - |
| (b2) | Pentaerythrityl tetra-(benzoate/2-ethyl-hexanoate) 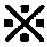 10 | - | - | - | - | - | - | - | 30 |
| (c) | Polyethylene wax | 8 | 8 | 8 | 8 | 8 | 8 | 8 | 8 |
| (d) | Dynamite glycerin | 6 | 6 | 6 | 6 | 6 | 6 | 6 | 6 |
| | Coloring material | 6 | 6 | 6 | 6 | 6 | 6 | 6 | 6 |
| | Hydrophobized pearlescent agent | 1 | 1 | 1 | 1 | 1 | 1 | 1 | 1 |
| Evaluation (1): Secondary adhesion resistance effect | | S | A | S | S | S | S | - | s |
| Evaluation (2): Stability | | A* | A* | A* | A* | A* | A | C | A |
| Evaluation (3): Gloss | | A* | A* | A* | A* | A* | A* | - | A* |
| Evaluation (4): Separation state of (a) and (b1) | | A | A | A | A | A | A | C | A |
| Relative amount of diphenylsiloxy phenyl trimethicone in component (b1) 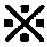 11 | | 3.33 | 3.33 | 1.6 | 1.6 | 1.6 | 0.86 | 0.44 | 0.4 |

| | | | | | | | | | |
|---|---|---|---|---|---|---|---|---|---|
| *(not according to the invention) | | | | | | | | | |

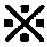 10: benzoate/2-ethylhexanoate = 2.5 mol/1.5 mol
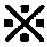 11: The value of diphenylsiloxy phenyl trimethicone/{other component (b1) other than diphenylsiloxy phenyl trimethicone} (mass ratio).

Here, the evaluation (4) in Test Example 3-7 indicates the evaluation results for the separation state of component (a) and component (b) ((b1) + (b2)).

According to Test Example 3-1 to Test Example 3-6, wherein the percentage of diphenylsiloxy phenyl trimethicone in component (b1) was varied, the stability was found to decrease with a decrease in the percentage.

In the sample of Test Example 3-6, wherein the relative amount of diphenylsiloxy phenyl trimethicone in component (b1) is 0.44, the stability was very poor.

According to Test Example 3-7, even when diphenylsiloxy phenyl trimethicone in component (b1) is 0.4, it was clarified that a lip cosmetic having good stability could be obtained by blending pentaerythrityl tetra(benzoate/2-ethylhexanoate). In addition, this sample was excellent in the secondary adhesion resistance effect.

Accordingly, in the case that pentaerythrityl tetra(benzoate/2-ethylhexanoate) isn't blended, it is required that the blending quantity of diphenylsiloxy phenyl trimethicone in component (b1), as defined by the ratio diphenylsiloxy phenyl trimethicone/{other component (b1) other than diphenylsiloxy phenyl trimethicone}, is 0.8 to 3.5 (mass ratio).

When pentaerythrityl tetra(benzoate/2-ethylhexanoate) is blended, it is preferable that diphenylsiloxy phenyl trimethicone/{other component (b1) other than diphenylsiloxy phenyl trimethicone} = 0.4 to 3.5 (mass ratio).

**[Table 41**

| Test Example | | 4-1 | 4-2 | 4-3 | 4-4 | 4-5 |
|---|---|---|---|---|---|---|
| (a) | Polyglyceryl-5 triisostearate 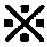 1 | 14 | 14 | 14 | 14 | 14 |
| (b1) | Diphenylsiloxy phenyl trimethicone 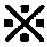 7 | 20 | 20 | 20 | 25 | 20 |
| | Diphenyl dimethicone 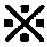 9 | 15 | 15 | 15 | 15 | 20 |
| (b2) | Pentaerythrityl tetra-(behenate/benzoate/2-ethylhexanoate) 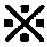 12 | 30 | - | - | 16 | 16 |
| | Dipentaerythrityl hexa-12-hydroxystearate | - | 30 | - | 9 | 9 |
| | Pentaerythrityl tetra-2-ethylhexanoate | - | - | 30 | - | - |
| (c) | Polyethylene wax | 8 | 8 | 8 | 8 | 8 |
| (d) | Dynamite glycerin | 6 | 6 | 6 | 6 | 6 |
| | Coloring material | 6 | 6 | 6 | 6 | 6 |
| | Hydrophobized pearlescent agent | 1 | 1 | 1 | 1 | 1 |
| Evaluation (1): Secondary adhesion resistance effect | | B* | A* | S | A* | S |
| Evaluation (2): Stability | | A* | A | A* | A* | A* |
| Evaluation (3): Gloss | | A* | A* | B* | S | S |

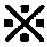 12: behenate/benzoate/2-ethylhexanoate = 1mol/2mol/1mol

According to Table 4, it is seen that a lip cosmetic having very good stability can also be obtained by blending an oil such as pentaerythrityl tetra(behenate/benzoate/2-ethylhexanoate), dipentaerythrityl hexa-12-hydroxystearate, or pentaerythrityl tetra-2-ethylhexanoate instead of pentaerythrityl tetra(benzoate/2-ethylhexanoate).

Accordingly, it is necessary to blend (b2) pentaerythritol ester in addition to component (a) and component (b1) in the lip cosmetic of the present invention. However, when the percentage of diphenylsiloxy phenyl trimethicone in component (b1) is large, as in Test Example 3-1, component (b2) does not have to be blended.

**[Table 5]**

| Test Example | | 5-1 | 5-2 | 3-7 | 5-3 | 5-4 | 5-5 | 5-6 | 5-7 |
|---|---|---|---|---|---|---|---|---|---|
| (a) | Polyglyceryl-5 triisostearate 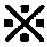 1 | 20 | 14 | 14 | 14 | 14 | 14 | 14 | 14 |
| (b1) | Diphenylsiloxy phenyl trimethicone 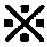 7 | - | 10 | 10 | 10 | 20 | 20 | 30 | 30 |
| | Trimethyl pentaphenyl trisiloxane 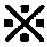 8 | 35 | 35 | 25 | 15 | 25 | 15 | 15 | - |
| | Diphenyl dimethicone 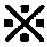 9 | - | - | - | - | - | - | - | 15 |
| (b2) | Pentaerythrityl tetra-(benzoate/2-ethylhexanoate) 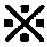 10 | 30 | 20 | 30 | 40 | 20 | 30 | 20 | 20 |
| (c) | Polyethylene wax | 8 | 8 | 8 | 8 | 8 | 8 | 8 | 8 |
| (d) | Dynamite glycerin | - | 6 | 6 | 6 | 6 | 6 | 6 | 6 |
| | Coloring material | 6 | 6 | 6 | 6 | 6 | 6 | 6 | 6 |
| | Hydrophobized pearlescent agent | 1 | 1 | 1 | 1 | 1 | 1 | 1 | 1 |
| Evaluation (1): Secondary adhesion resistance effect | | A* | S | S | A* | S | S | S | S |
| Evaluation (2): Stability | | A* | A | A | A | A* | A* | A* | A* |
| Evaluation (3): Gloss | | A* | A* | A* | A* | A* | A* | A* | A* |
| Relative amount of diphenylsiloxy phenyl trimethicone in component (b1) 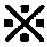 11 | | 0 | 0.29 | 0.4 | 0.67 | 0.8 | 1.33 | 2 | 2 |

Samples of Test Examples 5-1 to 5-7 and 3-7, wherein component (a), component (b1), and component (b2) were suitably blended, were excellent in the secondary adhesion resistance effect, and the stability and gloss were also excellent.

According to Table 5, in order to further improve the secondary adhesion resistance effect and stability, it is preferable that the relative amount of diphenylsiloxy trimethicone in component (b1) is 0.8 or higher.

**[Table 6]**

| Test Example | | 6-1* | 6-2* | 6-3* | 6-4 | 5-6 | 6-5 | 6-6 | 6-7 |
|---|---|---|---|---|---|---|---|---|---|
| (a) | Polyglyceryl-5 triisostearate 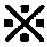 1 | 14 | 14 | 14 | 14 | 14 | 14 | 14 | 14 |
| (b1) | Diphenylsiloxy phenyl trimethicone 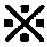 7 | - | 5 | 10 | 15 | 20 | 25 | 30 | 37.1 |
| | Trimethyl pentaphenyl trisiloxane 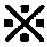 8 | - | 3.75 | 7.5 | 11.25 | 15 | 18.75 | 22.5 | 27.9 |
| (b2) | Pentaerythrityl tetra-(benzoate/2-ethyl-hexanoate) 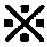 10 | 65 | 56.25 | 47.5 | 38.75 | 30 | 21.25 | 12.5 | - |
| (c) | Polyethylene wax | 8 | 8 | 8 | 8 | 8 | 8 | 8 | 8 |
| (d) | Dynamite glycerin | 6 | 6 | 6 | 6 | 6 | 6 | 6 | 6 |
| | Coloring material | 6 | 6 | 6 | 6 | 6 | 6 | 6 | 6 |
| | Hydrophobized pearlescent agent | 1 | 1 | 1 | 1 | 1 | 1 | 1 | 1 |
| Evaluation (1): Secondary adhesion resistance effect | | A | A | A* | A* | S | S | S | S |
| Evaluation (2): Stability | | C | C | B | A* | A* | A* | A* | A* |
| Evaluation (3): Gloss | | S | S | S | A* | A* | A* | A* | A* |
| Evaluation (4): Separation state of (a) and (b1) | | A | A | A | A | A | A | A | A |
| Relative amount of diphenyl-siloxy phenyl trimethicone in component (b1) 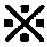 11 | | - | 1.33 | 1.33 | 1.33 | 1.33 | 1.33 | 1.33 | 1.33 |

| | | | | | | | | | |
|---|---|---|---|---|---|---|---|---|---|
| *(not according to the invention) | | | | | | | | | |

According to Table 6, in Test Examples 6-1 to 6-3, wherein the blending quantity of component (b1) was small, the sample stability was poor.

Accordingly, it is necessary that the blending quantity of (b1) methyl phenyl silicones that are blended in the lip cosmetic of the present invention is 20 mass % or higher and that the blending quantity of (b2) pentaerythritol ester is 45 mass % or lower.

**[Table 7]**

| Test Example | | 7-1 | 7-2 | 7-3 | 7-4 |
|---|---|---|---|---|---|
| (a) | Polyglyceryl-5 triisostearate 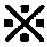 1 | 14 | 14 | 14 | 14 |
| (b1) | Diphenylsiloxy phenyl trimethicone 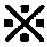 7 | 25 | 20 | 24.5 | 19.5 |
| | Diphenyl dimethicone 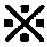 9 | 15 | 20 | 15 | 20 |
| (b2) | Pentaerythrityl tetra-(behenate/benzoate/2-ethylhexanoate) 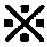 12 | 16 | 16 | 16 | 16 |
| | Dipentaerythrityl hexa-12-hydroxystearate | 9 | 9 | 9 | 9 |
| (c) | Polyethylene wax | 8 | 8 | - | - |
| | (Paraffin/microcrystalline wax) mixture 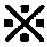 13 | - | - | 4.5 | 4.5 |
| | Candelilla wax | - | - | 4 | 4 |
| (d) | Dynamite glycerin | 6 | 6 | 6 | 6 |
| | Coloring material | 6 | 6 | 6 | 6 |
| | Hydrophobized pearlescent agent | 1 | 1 | 1 | 1 |
| Evaluation (1): Secondary adhesion resistance effect | | A* | A* | A* | A* |
| Evaluation (2): Stability | | A* | A* | A* | A* |
| Evaluation (3): Gloss | | S | S | S | S |

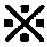 13: Paramix 91 (manufactured by NIKKO RICA CORPORATION)

According to Table 7, it is seen that various kinds of wax can be used as blendable (c) wax in the lip cosmetic of the present invention.

Hereinafter, Formulation Examples of the lip cosmetic of the present invention will be illustrated. It is to be understood that the present invention is not limited by these Formulation Examples and is specified by the scope of claims.

**[Table 8]**

| | | Formulation Example 1 |
|---|---|---|
| (a) | Polyglyceryl-5 triisostearate 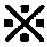 1 | 14 |
| (b1) | Diphenylsiloxy phenyl trimethicone 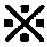 7 | 20 |
| | Trimethyl pentaphenyl trisiloxane 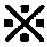 8 | 35 |
| (b2) | Pentaerythrityl tetra(benzoate/2-ethylhexanoate) 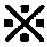 10 | 10 |
| (c) | Polyethylene wax | 8 |
| (d) | Dynamite glycerin | 6 |
| | Coloring material | 6 |
| | Hydrophobized pearlescent agent | 1 |
| Evaluation (1): Secondary adhesion resistance effect | | S |
| Evaluation (2): Stability | | A* |
| Evaluation (3): Gloss | | B* |
| Relative amount of diphenylsiloxy phenyl trimethicone in component (b1) 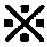 11 | | 0.57 |

**[Table 91**

| | | Formulation Example 2 | Formulation Example 3 | Formulation Example 4 |
|---|---|---|---|---|
| (a) | Polyglyceryl-5 triisostearate 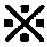 1 | 14 | 14 | 14 |
| (b1) | Diphenylsiloxy phenyl trimethicone 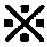 7 | 20 | 20 | 10 |
| | Diphenyl dimethicone 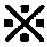 9 | 25 | 25 | 25 |
| (b2) | Dipentaerythrityl hexa-12-hydroxystearate | 20 | - | - |
| | Pentaerythrityl tetra-(behenate/benzoate/2-ethylhexanoate) 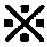 12 | - | 20 | - |
| | Pentaerythrityl tetra-2-ethylhexanoate | - | - | 30 |
| (c) | Polyethylene wax | 8 | 8 | 8 |
| (d) | Dynamite glycerin | 6 | 6 | 6 |
| | Coloring material | 6 | 6 | 6 |
| | Hydrophobized pearlescent agent | 1 | 1 | 1 |
| Evaluation (1): Secondary adhesion resistance effect | | S | A* | S |
| Evaluation (3): Gloss | | A* | A* | B* |
| Relative amount of diphenylsiloxy phenyl trimethicone in component (b1)11 | | 0.8 | 0.8 | 0.4 |

All the lipsticks shown in the following Formulation Examples 5 to 7 were excellent in the secondary adhesion resistance effect, stability, and gloss.

**Formulation Example 5: lipstick**

| | |
|---|---|
| Polyglyceryl-10 triisostearate | 14 mass % |
| Diphenylsiloxy phenyl trimethicone ( 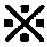 7) | 40 |
| Trimethyl pentaphenyl trisiloxane ( 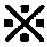 8) | 12.5 |
| Diphenyl dimethicone ( 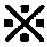 9) | 12.5 |
| Polyethylene wax | 8 |
| Glycerin | 6 |
| Coloring material | 6 |
| Hydrophobized pearlescent agent | 1 |

**Formulation Example 6: lipstick**

| | |
|---|---|
| Polyglyceryl-5 triisostearate | 14 mass % |
| Diphenylsiloxy phenyl trimethicone ( 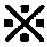 7) | 10 |
| Trimethyl pentaphenyl trisiloxane ( 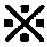 8) | 15 |
| Pentaerythrityl tetra(behenate/benzoate/2-ethylhexanoate) ( 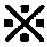 12) | 40 |
| Polyethylene wax | 8 |
| Glycerin | 6 |
| Coloring material | 6 |
| Hydrophobized pearlescent agent | 1 |

**Formulation Example 7: lipstick**

| | |
|---|---|
| Polyglyceryl-5 tetraisostearate | 14 mass % |
| Diphenylsiloxy phenyl trimethicone ( 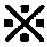 7) | 40 |
| Trimethyl pentaphenyl trisiloxane ( 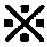 8) | 12.5 |
| Diphenyl dimethicone ( 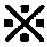 9) | 12.5 |
| Polyethylene wax | 8 |
| Glycerin | 6 |
| Coloring material | 6 |
| Hydrophobized pearlescent agent | 1 |

## Claims

1. A lip cosmetic comprising the following (a) and (b), wherein the blending quantity of (b) is 20 to 70 mass %:
(a) 5 to 20 mass % of a polyglyceryl isostearate wherein the addition mole number of glycerin is 4 to 10, and the number of isostearic acid residues is 1 to 4; and
(b)
(b1) 20 to 70 mass % of one or more kinds of methyl phenyl silicones, wherein said one or more kinds of methyl phenyl silicones are selected from the group consisting of trimethyl pentaphenyl trisiloxane, diphenyl dimethicone and diphenylsiloxy phenyl trimethicone,
(b2) 0 to 45 mass % of pentaerythrityl tetra(benzoate/2-ethylhexanoate) wherein, when component (b1) contains diphenylsiloxy phenyl trimethicone and component (b2) is absent, the blending quantity of diphenylsiloxy phenyl trimethicone in component (b1), as defined by the equation diphenylsiloxy phenyl trimethicone/{component (b1) other than diphenylsiloxy phenyl trimethicone}, is 0.8 to 3.5 (mass ratio).

2. The lip cosmetic according to claim 1, wherein component (b1) as a whole does not separate when mixed with component (a) at 130 °C and separates when mixed with component (a) at 25 °C.

3. The lip cosmetic according to claim 1 or 2, wherein (c) 4 to 10 mass % of a wax is contained.

4. The lip cosmetic according to any of claims 1 to 3, wherein component (b1) contains diphenylsiloxy phenyl trimethicone.

5. The lip cosmetic according to claim 4, wherein, when component (b2) is present, the blending quantity of diphenylsiloxy phenyl trimethicone in component (b1), as defined by the equation diphenylsiloxy phenyl trimethicone/{other component (b1) other than diphenylsiloxy phenyl trimethicone}, is 0.4 to 3.5 (mass ratio).

6. The lip cosmetic according to any of claims 1 to 5, wherein (d) glycerin is contained.

7. The lip cosmetic according to any of claims 1 to 6, wherein component (a) contains 40 mass % or higher polyglyceryl isostearate, which has a degree of polymerization of 5, in the total amount of component (a).

8. The lip cosmetic according to any of claims 1 to 7, wherein component (a) is polyglyceryl-5 triisostearate.

9. A lip cosmetic comprising the following (a) and (b), wherein the blending quantity of (b) is 30 to 70 mass %:
(a) 5 to 20 mass % of a polyglyceryl isostearate wherein the addition mole number of glycerin is 4 to 10, and the number of isostearic acid residues is 1 to 4; and
(b)
(b1) 20 to 60 mass % of one or more kinds of methyl phenyl silicones, wherein said one or more kinds of methyl phenyl silicones are selected from the group consisting of trimethyl pentaphenyl trisiloxane, diphenyl dimethicone and diphenylsiloxy phenyl trimethicone,
(b2) 5 to 45 mass % of a pentaerythritol ester.

10. A lip cosmetic comprising the following (a) to (c):
(a) 5 to 20 mass % of a polyglyceryl isostearate wherein the addition mole number of glycerin is 4 to 10, and the number of isostearic acid residues is 1 to 4;
(b) 20 to 70 mass % of one or more kinds of methyl phenyl silicones that does not separate when mixed with component (a) at 130 °C and separate when mixed with component (a) at 25 °C, wherein said one or more kinds of methyl phenyl silicones are selected from the group consisting of trimethyl pentaphenyl trisiloxane, diphenyl dimethicone and diphenylsiloxy phenyl trimethicone; and
(c) 5 to 10 mass % of a wax,
wherein, when component (b) contains diphenylsiloxy phenyl trimethicone and the lip cosmetic does not comprise pentaerythrityl tetra(benzoate/2-ethylhexanoate) the blending quantity of diphenylsiloxy phenyl trimethicone in component (b), as defined by the equation diphenylsiloxy phenyl trimethicone/{component (b) other than diphenylsiloxy phenyl trimethicone}, is 0.8 to 3.5 (mass ratio).

## Patentansprüche

1. Lippenkosmetikum, umfassend die nachfolgenden Komponenten (a) und (b), wobei die eingemischte Menge an (b) 20 bis 70 Masse% beträgt:
(a) 5 bis 20 Masse% eines Polyglycerylisostearats, wobei die Molzahl an zugegebenem Glycerin 4 bis 10 beträgt und die Anzahl an Isostearinsäureresten 1 bis 4 beträgt; und
(b)
(b1) 20 bis 70 Masse% einer Art oder mehrerer Arten von Methylphenylsilikonen, wobei die eine Art oder die mehreren Arten von Methylphenylsilikonen aus der Gruppe bestehend aus Trimethylpentaphenyltrisiloxan, Diphenyldimethicon und Diphenylsiloxyphenyltrimethicon ausgewählt ist/sind,
(b2) 0 bis 45 Masse% an Pentaerythrityltetra(benzoat/2-ethylhexanoat),
wobei in Fällen, in welchen Komponente (b1) Diphenylsiloxyphenyltrimethicon enthält und Komponente (b2) abwesend ist, die in Komponente (b1) eingemischte Menge an Diphenylsiloxyphenyltrimethicon, wie sie durch die Gleichung Diphenylsiloxyphenyltrimethicon/{Komponente (b1) mit Ausnahme von Diphenylsiloxyphenyltrimethicon} definiert ist, 0.8 bis 3.5 (Massenverhältnis) beträgt.

2. Lippenkosmetikum nach Anspruch 1, wobei sich Komponente (b1) in ihrer Gesamtheit bei 130°C aus einem Gemisch mit Komponente (a) nicht absondert und sich bei 25°C aus einem Gemisch mit Komponente (a) absondert.

3. Lippenkosmetikum nach Anspruch 1 oder 2, wobei (c) 4 bis 10 Masse% eines Wachses enthalten sind.

4. Lippenkosmetikum nach einem der Ansprüche 1 bis 3, wobei Komponente (b1) Diphenylsiloxyphenyltrimethicon enthält.

5. Lippenkosmetikum nach Anspruch 4, wobei in Fällen, in welchen Komponente (b2) anwesend ist, die in Komponente (b1) eingemischte Menge an Diphenylsiloxyphenyltrimethicon, wie sie durch die Gleichung Diphenylsiloxyphenyltrimethicon/{Komponente (b1) mit Ausnahme von Diphenylsiloxyphenyltrimethicon) definiert ist, 0.4 bis 3.5 (Massenverhältnis) beträgt.

6. Lippenkosmetikum nach einem der Ansprüche 1 bis 5, wobei (d) Glycerin enthalten ist.

7. Lippenkosmetikum nach einem der Ansprüche 1 bis 6, wobei Komponente (a) 40 Masse% oder mehr an Polyglycerylisostearat mit einem Polymerisationsgrad von 5, bezogen auf die Gesamtmenge an Komponente (a), enthält.

8. Lippenkosmetikum nach einem der Ansprüche 1 bis 7, wobei es sich bei Komponente (a) um Polyglyceryl-5-triisostearat handelt.

9. Lippenkosmetikum, umfassend die nachfolgenden Komponenten (a) und (b), wobei die eingemischte Menge an (b) 30 bis 70 Masse% beträgt:
(a) 5 bis 20 Masse% eines Polyglycerylisostearats, wobei die Molzahl an zugegebenem Glycerin 4 bis 10 beträgt und die Anzahl an Isostearinsäureresten 1 bis 4 beträgt; und
(b)
(b1) 20 bis 60 Masse% einer Art oder mehrerer Arten von Methylphenylsilikonen, wobei die eine Art oder die mehreren Arten von Methylphenylsilikonen aus der Gruppe bestehend aus Trimethylpentaphenyltrisiloxan, Diphenyldimethicon und Diphenylsiloxyphenyltrimethicon ausgewählt ist/sind,
(b2) 5 bis 45 Masse% eines Pentaerythritolesters.

10. Lippenkosmetikum, umfassend die nachfolgenden Komponenten (a) bis (c):
(a) 5 bis 20 Masse% eines Polyglycerylisostearats, wobei die Molzahl an zugegebenem Glycerin 4 bis 10 beträgt und die Anzahl an Isostearinsäureresten 1 bis 4 beträgt;
(b) 20 bis 70 Masse% einer Art oder mehrerer Arten von Methylphenylsilikonen, welche sich bei 130°C aus einem Gemisch mit Komponente (a) nicht absondern und sich bei 25°C aus einem Gemisch mit Komponente (a) absondern, wobei die eine Art oder die mehreren Arten von Methylphenylsilikonen aus der Gruppe bestehend aus Trimethylpentaphenyltrisiloxan, Diphenyldimethicon und Diphenylsiloxyphenyltrimethicon ausgewählt ist/sind; und
(c) 5 bis 10 Masse% eines Wachses,
wobei in Fällen, in welchen Komponente (b) Diphenylsiloxyphenyltrimethicon enthält und das Lippenkosmetikum kein Pentaerythrityltetra(benzoat/2-ethylhexanoat) umfasst, die in Komponente (b) eingemischte Menge an Diphenylsiloxyphenyltrimethicon, wie sie durch die Gleichung Diphenylsiloxyphenyltrimethicon/{Komponente (b1) mit Ausnahme von Diphenylsiloxyphenyltrimethicon} definiert ist, 0.8 bis 3.5 (Massenverhältnis) beträgt.

## Revendications

1. Cosmétique pour les lèvres comprenant les éléments (a) et (b) suivants, dans lequel la quantité de mélange de (b) est de 20 à 70 % en masse :
(a) 5 à 20 % en masse d'un isostéarate de polyglycéryle dans lequel le nombre de mole d'addition de glycérine est de 4 à 10, et le nombre de résidus acide isostéarique est de 1 à 4 ; et
(b)
(b1) 20 à 70 % en masse d'un ou plusieurs types de méthyl phényl silicones, dans lequel lesdits un ou plusieurs types de méthyl phényl silicones sont choisis dans le groupe consistant en le triméthyl pentaphényl trisiloxane, la diphényl diméthicone et la diphénylsiloxy phényl triméthicone,
(b2) 0 à 45 % en masse de tétra(benzoate/2-éthylhexanoate) de pentaérythrityle,
dans lequel, lorsque le composant (b1) contient de la diphénylsiloxy phényl triméthicone et le composé (b2) est absent, la quantité de mélange de diphénylsiloxy phényl triméthicone dans le composant (b1), telle que définie par l'équation diphénylsiloxy phényl triméthicone/{composant (b1) autre que diphénylsiloxy phényl triméthicone}, est de 0,8 à 3,5 (rapport en masse).

2. Cosmétique pour les lèvres selon la revendication 1, dans lequel le composant (b1) dans son ensemble ne se sépare pas lorsqu'il est mélangé avec le composant (a) à 130 °C et se sépare lorsqu'il est mélangé avec le composant (a) à 25 °C.

3. Cosmétique pour les lèvres selon la revendication 1 ou 2, dans lequel (c) 4 à 10 % en masse d'une cire sont contenus.

4. Cosmétique pour les lèvres selon l'une quelconque des revendications 1 à 3, dans lequel le composant (b1) contient de la diphénylsiloxy phényl triméthicone.

5. Cosmétique pour les lèvres selon la revendication 4, dans lequel, lorsque le composant (b2) est présent, la quantité de mélange de diphénylsiloxy phényl triméthicone dans le composant (b1), telle que définie par l'équation diphénylsiloxy phényl triméthicone/{autre composant (b1) autre que diphénylsiloxy phényl triméthicone}, est de 0,4 à 3,5 (rapport en masse).

6. Cosmétique pour les lèvres selon l'une quelconque des revendications 1 à 5, dans lequel (d) de la glycérine est contenue.

7. Cosmétique pour les lèvres selon l'une quelconque des revendications 1 à 6, dans lequel le composant (a) contient 40 % en masse ou plus d'isostéarate de polyglycéryle, qui a un degré de polymérisation de 5, dans la quantité totale de composant (a).

8. Cosmétique pour les lèvres selon l'une quelconque des revendications 1 à 7, dans lequel le composant (a) est le triisostéarate de polyglycéryle-5).

9. Cosmétique pour les lèvres comprenant les éléments (a) et (b) suivants, dans lequel la quantité de mélange de (b) est de 30 à 70 % en masse :
(a) 5 à 20 % en masse d'un isostéarate de polyglycéryle dans lequel le nombre de mole d'addition de glycérine est de 4 à 10, et le nombre de résidus acide isostéarique est de 1 à 4 ; et
(b)
(b1) 20 à 60 % d'un ou plusieurs types de méthyl phényl silicones, dans lequel lesdits un ou plusieurs types de méthyl phényl silicones sont choisis dans le groupe consistant en le triméthyl pentaphényl trisiloxane, la diphényl diméthicone et la diphénylsiloxy phényl triméthicone,
(b2) 5 à 45 % en masse d'un ester de pentaérythritol.

10. Cosmétique pour les lèvres comprenant les éléments (a) à (c) suivants :
(a) 5 à 20 % en masse d'un isostéarate de polyglycéryle dans lequel le nombre de mole d'addition de glycérine est de 4 à 10, et le nombre de résidus acide isostéarique est de 1 à 4 ;
(b) 20 à 70 % en masse d'un ou plusieurs types de méthyl phényl silicones qui ne se séparent pas lorsqu'ils sont mélangés avec le composant (a) à 130 °C et se séparent lorsqu'ils sont mélangés avec le composant (a) à 25 °C, dans lequel lesdits un ou plusieurs types de méthyl phényl silicones sont choisis dans le groupe consistant en le triméthyl pentaphényl trisiloxane, la diphényl diméthicone et la diphénylsiloxy phényl triméthicone ; et
(c) 5 à 10 % en masse d'une cire,
dans lequel, lorsque le composant (b) contient de la diphénylsiloxy phényl triméthicone et que le cosmétique pour les lèvres ne comprend pas de tétra(benzoate/2-éthylhexanoate) de pentaérythrityle, la quantité de mélange de diphénylsiloxy phényl triméthicone dans le composant (b), telle que définie par l'équation diphénylsiloxy phényl triméthicone/{composant (b) autre que diphénylsiloxy phényl triméthicone}, est de 0,8 à 3,5 (rapport en masse).
